Europäisches Patentamt

(19) European Patent Office

Office européen des brevets

(11) Publication number : **0 403 303 B1**

# EUROPEAN PATENT SPECIFICATION

(45) Date of publication of patent specification :
**23.03.94 Bulletin 94/12**

(51) Int. Cl.⁵ : **A61K 7/06**

(21) Application number : **90306563.9**

(22) Date of filing : **15.06.90**

(54) Hair treatment composition.

(30) Priority : **16.06.89 GB 8913879**

(43) Date of publication of application :
**19.12.90 Bulletin 90/51**

(45) Publication of the grant of the patent :
**23.03.94 Bulletin 94/12**

(84) Designated Contracting States :
**AT BE CH DE DK ES FR GB GR IT LI NL SE**

(56) References cited :
EP-A- 0 007 785
EP-A- 0 086 070
EP-A- 0 232 982
EP-A- 0 261 812
US-A- 4 380 549
CHEMICAL ABSTRACTS, vol. 110, no. 12, 20
March 1989 Columbus, Ohio, USA J.C. Hill et
al.: "The skin plasticization effect of a medium
chain 2-hydroxy acid and the use of poten-
tiators" page 406; column 1; ref. no. 101521R
& J. Appl. Cosmetol. 1988,6(2),pages 53-68

(56) References cited :
CHEMICAL ABSTRACTS, vol. 101, no. 2, 9 July
1984 Columbus, Ohio, USA Susan g. Alderson
et al.: "Effect of 2-hydroxyacids on guinea pig
footpad stratum corneum:mechanical proper-
ties and binding studies" page 288; column 2;
ref. no. 12004W & Int. J. Cosmet. Sci.
1984,6(2),pages 91-100

(73) Proprietor : **UNILEVER PLC**
**Unilever House Blackfriars**
**London EC4P 4BQ (GB)**
(84) **GB**
Proprietor : **UNILEVER N.V.**
**Weena 455**
**NL-3013 AL Rotterdam (NL)**
(84) **BE CH DE DK ES FR GR IT LI NL SE AT**

(72) Inventor : **Gallagher, Peter**
**1 Mission Cottage, (Moss Lane)**
**Burscough, Lancashire (GB)**
Inventor : **McGee, Thomas**
**74 Stanly Road**
**Hoylake, Wirral, Merseyside L47 1H7 (GB)**

(74) Representative : **Tonge, Robert James et al**
**UNILEVER PLC Patent Division Colworth**
**House Sharnbrook**
**Bedford MK44 1LQ (GB)**

## Description

FIELD OF INVENTION

The invention relates to hair conditioning compositions containing 2-hydroxyalkanoic acids.

BACKGROUND OF THE INVENTION

Certain 2-hydroxyalkanoic acids are known for their skin benefits when included in compositions for topical application to the skin. Such benefits include both increased extensibility and improved appearance.

Hair which has reduced elasticity, as a result of ageing or of chemical or mechanical treatment regimes is more likely to fracture when subjected to mechanical stress. By increasing hair elasticity, the hair becomes not only more resistant to such stress, but also more appealing cosmetically. The hair feels softer and is less flyaway.

Hydroxycarboxylic acids have previously been included in skin creams where they have been shown to give some benefit in the treatment of various skin disorders. - Examples of such skin creams can be found in US 3 920 835, US 3 984 566 or US 4 363 815, all in the names of Van Scott and Yu.

Skin creams containing a combination of 2-hydroxy octanoic acid and alkyl lactate are disclosed in US 4 507 319 (Unilever), and are said to be particularly effective against acne.

EP 7785 (Unilever) relates to skin compositions containing hydroxylated $C_6$ to $C_{10}$ carboxylic acids which can be formulated with a number of vehicles including cationic emulsifiers. These compositions contain no co-acid buffer to maintain an acid pH.

The use of 2-hydroxy octanoic acid in the treatment of dandruff and of excessively dry scalp with defective hair growth is disclosed in EP 232 982 (Unilever), in which the acid is applied to the hair in a composition which also comprises nonionic surfactant. Nonionic surfactant is preferred in those applications, to minimise exacerbation of the skin conditions.

DE 2 110 993 (Henkel) relates to liquid cleaning compositions which comprise alkali metal salts of hydroxycarboxylic acids. These compositions contain no co-acid buffer to maintain an acid pH.

It has now been found surprisingly that when certain 2-hydroxyalkanoic acids are incorporated into hair conditioning compositions, they increase the elasticity of the hair, thus producing a cosmetic benefit.

The effect can be seen on application of 2-hydroxyalkanoic acids having chain lengths of $C_4$ to $C_{20}$.

BRIEF SUMMARY OF THE INVENTION

The invention provides a method of enhancing the elasticity of hair, comprising applying thereto an aqueous hair conditioning composition comprising

(a) from 0.1 to 20 % by weight of a 2-hydroxyalkanoic acid chosen from 2-hydroxyhexanoic acid, 2-hydroxyoctanoic acid, 2-hydroxydecanoic acid or mixtures thereof;

(b) from 0.01 to 10% by weight of a conditioning agent; and

(c) from 0.1 to 10% by weight of a co-acid buffering agent.

The invention also provides, in another aspect, the use of the above-defined compositions for enhancing the elasticity of hair.

DETAILED DESCRIPTION OF INVENTION

The 2-hydroxyalkanoic acid

The hair conditioning composition of the invention contains from 0.1 to 20% by weight, preferably from 1 to 10% by weight of a 2-hydroxyalkanoic acid, chosen from 2-hydroxyhexanoic acid, 2-hydroxyoctanoic acid, 2-hydroxydecanoic acid or mixtures thereof. The most preferred acid is 2-hydroxyoctanoic acid.

If less than 0.1% by weight of 2-hydroxyalkanoic acid is included in the composition, little increase in elasticity of the hair is observed, and if more than 20% by weight is included, no added benefit is seen.

Conditioning agent

The hair conditioning composition of the invention also comprises a conditioning agent. The conditioning agent is preferably chosen from cationic surfactants, cationic polymers, quaternised silicones, volatile silicones, protein hydrolysates or quaternised protein hydrolysates.

Examples of cationic surfactants include

Cetyl trimethylammonium chloride

Stearyl dimethylbenzyl ammonium chloride

Cetylpyridinium chloride

Quaternium -5

Quaternium -31

Quaternium -18

and mixtures thereof

Suitable cationic polymers include

Guar Hydroxypropyltrimonium chloride

Quaternium -19

Quaternium -23

Quaternium -40

Quaternium -57

Poly(dimethyldiallyammonium chloride) Poly(dimethylbutenyl ammonium chloride)-$\alpha,\omega$-bis(triethano-lammonium chloride)

Poly(dipropyldiallylammonium chloride)

Poly(methyl-$\beta$-propaniodiallylammonium chloride)

Poly(diallylpiperidinium chloride)

Poly(vinyl pyridinium chloride)

Quaternised poly (vinyl alcohol)

Quaternised poly (dimethylaminoethylmethacrylate) and mixtures thereof.

Examples of suitable volatile silicone materials include cyclomethicone available commercially as Dow Corning DC 345 and Volatile Silicone 7158, available from Union Carbide.

The quaternised silicones which may be used in the compositions of the invention are generally amino functional polydimethylsiloxanes. Examples include amodimethicone available commercially as Dow Corning DC 929 and trimethylsilylamodimethicone, available as Dow Corning Q 8220.

Suitable protein hydrolysates include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

The conditioning composition of the invention comprises from 0.01 to 10% by weight, preferably from 0.1 to 1% by weight of the conditioning agent.

## Co-acid buffering agent

The composition also comprises a co-acid buffering agent which acts to maintain a low pH. We have found that the hair can buffer a weak solution of 2-hydroxyalkanoic acid giving rise to the salt of the acid, which has a lower penetration than the acid itself.

Suitable co-acid buffering agents include lactic acid, citric acid tartaric acid, acetic acid, formic acid, malonic acid, glycolic acid, thioglycollic acid, benzoic acid, adipic acid, malic acid and mesaconic acid. The most preferred acid is lactic acid.

The co-acid buffering agent is present in the composition in an amount of 0.1 to 10%, preferably at least 1%, more preferably from 2 to 5% by weight. The total of 2-hydroxyalkanoic acid plus buffering agent will frequently be at least 3% by weight of the composition.

The pH of hair conditioning composition of the invention is preferably in the range of from 2 to 4.5.

## Other ingredients

The composition of the invention may also comprise a penetration enhancer which aids the penetration of materials into the hair. Suitable examples of penetration enhancers include butane-1,3-diol and urea.

The hair conditioning composition of the invention also comprises certain ingredients known in the art and necessary to the particular formulation. Examples of other ingredients include surfactants, viscosity control agents, solubility control agents, foam boosters, opacifiers, perfumes, colouring agents, antidandruff agents, preservatives, proteins, polymers, buffering agents, thickening agents such as hydroxyethyl cellulose, sold commercially as Natrosol* 250HR, hydroxypropyl guar, sold commercially as Jaguar* HP60, and xanthan gum, sold commercially as Kelzan*, and agents thought to have a beneficial effect on the condition of hair such as

* trade marks

vitamins, amino acids and trace elements.

PROCESS

The hair conditioning composition of the invention is formulated by mixing together the required ingredients in the amounts specified.

PRODUCT FORMS AND PACKAGING

The hair conditioning composition of the invention is commonly packaged in a bottle with lid or a dispenser with a pump. In general, the composition of the invention may be formulated in any manner that allows application to the hair so that the benefit conferred by the 2-hydroxyalkanoic acid on the hair is discernible. Possible packaging variants are those known to the person skilled in the field of hair treatment compositions.

The composition may be applied as pre-treatments to be used before shampooing, or as post-shampoo rinse-off or leave-on products.

ADVANTAGES OF THE INVENTION

The hair conditioning composition of the invention has been shown in tests involving Instron measurements of hair elasticity to increase the elasticity of hair significantly at the 95% confidence level. Although the applicant does not wish to be bound by theory, it is thought that there exists a correlation between the increase in elasticity of hair when treated with compositions of the invention and the degree of plasticisation of the hair protein chains. Pulsed NMR measurements confirm that plasticisation in hair treated with a composition containing a 2-hydroxyalkanoic acid is greater than in untreated hair. The effect is particularly marked in the case of hair that has been damaged chemically or mechanically.

Hair with increased elasticity tends to break less easily under mechanical stress such as brushing or combing. Negroid hair that has been treated with the composition of the invention becomes softer, more pliable, and more fracture-resistant than untreated hair. European and Thai hair show improved elasticity.

COMPARATIVE EXAMPLES

The following comparative examples illustrate the effect of 2-hydroxyalkanoic acid retention on hair properties.

Comparative Example 1

Hair switches were immersed in a 1% by weight aqueous solution of 2-hydroxyoctanoic acid (pH 3.0) for 24 hours. The hair: liquor ratio was 1:200 by weight. Instron tests showed that the increase in elasticity resulting from treatment with the 2-hydroxyoctanoic acid was statistically significant at the 95% confidence level as compared to virgin, untreated hair.

Comparative Example 2

Switches of Thai hair were soaked for 24 hours in 0.0625M solutions of 2-hydroxy alkanoic acids having chain lengths of $C_2$, $C_6$, $C_8$ and $C_{14}$, in a hair to liquor ratio of 1:200. The $C_{14}$ hydroxy acid is sparingly soluble in water so an ethanolic solution was used. Control results were obtained by soaking hair switches in water or ethanol (as control for $C_{14}$) for 24 hours.

It was found that all of the acids tested increase the elasticity of the hair with the 2-hydroxy octanoic acid giving the optimum effect.

Comparative Example 3

This comparative example illustrates the effect of a co-acid buffer on retention of 2-hydroxyalkanoic acid. Hair switches were treated with a composition containing 1% radiolabelled 2-hydroxyoctanoic acid and 2% lactic acid at a pH of 3.0. After several applications, the degree of retention of the 2-hydroxyoctanoic acid by the hair was measured and was found to be greater than for hair treated with a similar composition containing no lactic acid. This demonstrates that 2-hydroxyalkanoic acid retention by the hair is enhanced in the presence of a co-acid buffer.

Comparative Example 4

Hair switches (0.5g, 10 cm long) were washed. Conditioner composition, containing varying levels of 2-hydroxy octanoic acid and lactic acid as set out below, was applied to each switch, left for one minute, and the switch was rinsed for 30 seconds.

The 2-hydroxy octanoic acid was radiolabelled with $^{14}$C so that the amount of 2-hydroxy octanoic acid retained on the hair could be assesed.

The switch was blown dry, shampooed and the conditioner reapplied. The level of radioactivity retained on the hair was measured after 1, 5 and 10 treatments.

The conditioner compositions all had a pH of 3 and contained -

A. 1% by weight 2-hydroxyoctanoic acid
B. 1% by weight 2-hydroxyoctanoic acid and 2% by weight lactic acid
C. 2% by weight 2-hydroxyoctanoic and and 4% by weight lactic acid

The results were as follows. The measurements are given as g of 2-hydroxyoctanoic acid (x10$^{-4}$) retained per g of hair.

| No. applications | A | B | C |
| --- | --- | --- | --- |
| 1 | 2.15 | 3.03 | – |
| 5 | 3.12 | 7.55 | 16.5 |
| 10 | 4.08 | 11.1 | – |

g/g hair (x10$^{-4}$)

It can be seen that the level of 2-hydroxyoctanoic acid which is retained on the hair is significantly enhanced by the addition of lactic acid and by increasing the level of 2-hydroxyoctanoic acid in the composition.

The effect of successive treatments with a hair conditioner compositioner according to the invention is to build-up the level of 2-hydroxy alkanoic acid retained on the hair, giving increasing elasticity and hence increasing cosmetic benefit.

The following Examples illustrate conditioning compositions according to the invention. The ingredients are mixed together to form the composition, and the pH of each of the compositions is adjusted to 2.0 to 4.5 using a base, for example triethanolamine.

Example 1

| | % wt |
| --- | --- |
| Cetyl trimethyl ammonium chloride | 0.7 |
| Ceto/stearyl alcohol | 2.0 |
| Hydroxyoctanoic acid | 5.0 |
| Paraffin wax | 1.0 |
| Lactic acid | 1.0 |
| Glycerol monostearate | 0.7 |
| Preservative, perfume, colour | qs |
| Water | to 100 |

5

Example 2

|  | %wt |
|---|---|
| Cetyl trimethyl ammonium chloride | 0.7 |
| Ceto/stearyl alcohol | 1.0 |
| Hydroxyhexanoic acid | 5.5 |
| Natrosol 250HR | 1.3 |
| Acetic acid | 1.6 |
| Preservative, perfume, colour | qs |
| Water | to 100 |

Example 3

|  | %wt |
|---|---|
| Dicetyl dimethyl ammonium chloride | 0.4 |
| Jaguar HP60 (1) | 1.0 |
| Hydroxydecanoic acid | 2.4 |
| Malic acid | 0.8 |
| Preservative, perfume, colour | qs |
| Water | to 100 |

(1)   Jaguar HP60 is hydroxypropyl guar

Example 4

|  | % wt |
|---|---|
| Ethanol | 30 |
| Hydroxyhexanoic acid | 5.0 |
| Cetyl trimethyl ammonium chloride | 0.4 |
| Malic acid | 1.5 |
| Perfume, colour, preservative | qs |
| Water | to 100 |

Example 5

|  | % wt |
|---|---|
| Ethanol | 30 |
| Hydroxyoctanoic acid | 4.6 |
| Cetyl trimethyl ammonium chloride | 0.6 |
| Acetic acid | 1.0 |
| Perfume, colour, preservative | qs |
| Water | to 100 |

**Claims**

1.  A method of enhancing the elasticity of hair, comprising applying thereto an aqueous hair conditioning composition comprising:
    (a) from 0.1 to 20% by weight of a 2-hydroxyalkanoic acid chosen from 2-hydroxyhexanoic acid, 2-hydroxyoctanoic acid, 2-hydroxydecanoic acid or mixtures thereof;
    (b) from 0.01 to 10% by weight of a conditioning agent; and
    (c) from 0.1 to 10% by weight of a co-acid buffering agent.

2.  A method according to claim 1, wherein the 2-hydroxyalkanoic acid in the composition is 2-hydroxyoctanoic acid.

3.  A method according to claim 1 or claim 2, wherein the 2-hydroxyalkanoic acid is present in the composition in an amount from 1 to 10% by weight.

4.  A method according to any one of claims 1 to 3, wherein the conditioning agent in the composition is chosen from cationic surfactants, cationic polymers, quaternised silicones, volatile silicones, quaternised polymers, protein hydrolysates or quaternised protein hydrolysates.

5.  A method according to any preceding claim, wherein the conditioning agent in the composition is present in an amount of from 0.1 to 1% by weight.

6.  A method according to any preceding claim, wherein the co-acid buffering agent in the composition is lactic acid.

7.  A method according to any preceding claim, wherein the co-acid buffering agent in the composition is present in an amount from 2 to 5% by weight.

8.  A method according to to any preceding claim, wherein the total of 2-hydroxyalkanoic acid and co-acid buffering agent in the composition is at least 3% by weight.

9.  Use for enhancing the elasticity of hair of an aqueous hair conditioning composition which comprises:
    (a) from 0.1 to 20% by weight of a 2-hydroxyalkanoic acid chosen from 2-hydroxyhexanoic acid, 2-hydroxyoctanoic acid, 2-hydroxydecanoic acid or mixtures thereof;
    (b) from 0.01 to 10% by weight of a conditioning agent; and
    (c) from 0.1 to 10% by weight of a co-acid buffering agent.

**Patentansprüche**

1.  Verfahren zur Steigerung der Elastizität von Haar, wobei eine wäßrige Haarkonditionierungs-Zusammensetzung darauf aufgetragen wird, enthaltend:

(a) 0.1 bis 20 Gew.% einer 2-Hydroxyalkansäure, ausgewählt aus 2-Hydroxyhexansäure, 2-Hydroxyoctansäure, 2-Hydroxydecansäure oder Mischungen davon;
(b) 0.01 bis 10 Gew.% eines Konditionierungsmittels; und
(c) 0.1 bis 10 Gew.% eines Zusatzsäurepuffers.

2. Verfahren nach Anspruch 1, worin die 2-Hydroxyalkansäure in der Zusammensetzung 2-Hydroxyoctansäure ist.

3. Verfahren nach Anspruch 1 oder Anspruch 2, worin die 2-Hydroxyalkansäure in der Zusammensetzung in einer Menge von 1 bis 10 Gew.% vorhanden ist.

4. Verfahren nach einem der Ansprüche 1 bis 3, worin das Konditionierungsmittel in der Zusammensetzung ausgewählt ist aus kationischen Tensiden, kationischen Polymeren, quaternisierten Siliconen, flüchtigen Siliconen, quaternisierten Polymeren, Proteinhydrolysaten oder quaternisierten Proteinhydrolysaten.

5. Verfahren nach einem der vorstehenden Ansprüche, worin das Konditionierungsmittel in der Zusammensetzung in einer Menge von 0.1 bis 1 Gew.% vorhanden ist.

6. Verfahren nach einem der vorhergehenden Ansprüche, worin das Zusatzsäurepuffermittel in der Zusammensetzung Milchsäure ist.

7. Verfahren nach einem der vorhergehenden Ansprüche, worin das Zusatzsäurepuffermittel in der Zusammensetzung in einer Menge von 2 bis 5 Gew.% vorhanden ist.

8. Verfahren nach einem der vorhergehenden Ansprüche, worin die 2-Hydroxyalkansäure und das Zusatzsäurepuffermittel in der Zusammensetzung zusammen mindestens 3 Gew.% beträgt.

9. Verwendung einer wäßrigen Haarkonditionierungs-Zusammensetzung zur Steigerung der Haarelastizität, enthaltend:
(a) 0.1 bis 20 Gew.% einer 2-Hydroxyalkansäure, ausgewählt aus 2-Hydroxyhexansäure, 2-Hydroxyoctansäure, 2-Hydroxydecansäure oder Mischungen davon;
(b) 0.01 bis 10 Gew.% eines Konditionierungsmittels, und
(c) 0.1 bis 10 Gew.% eines Zusatzsäurepuffers.

## Revendications

1. Un procédé pour améliorer l'élasticité des cheveux comprenant le fait d'y appliquer une composition aqueuse de conditionnement des cheveux comprenant
(a) de 0,1 à 20 % en masse d'un acide 2-hydroxyalcanoïque choisi à partir de l'acide 2-hydroxyhexanoïque, de l'acide 2-hydroxyoctanoïque, de l'acide 2-hydroxydécanoïque ou de mélanges de ceux-ci;
(b) de 0,01 à 10 % en masse d'un agent de conditionnement; et
(c) de 0,1 à 10 % en masse d'un agent tampon co-acide.

2. Un procédé selon la revendication 1, dans lequel l'acide 2-hydroxyalcanoïque dans la composition est de l'acide 2-hydroxyoctanoïque.

3. Un procédé selon la revendication 1 ou la revendication 2, dans lequel l'acide 2-hydroxyalcanoïque est présent dans la composition dans une quantité allant de 1 à 10 % en masse.

4. Un procédé selon l'une des revendications 1 à 3, dans lequel l'agent de conditionnement dans la composition est choisi à partir des agents tensioactifs cationiques, des polymères cationiques, des silicones quaternisées, des silicones volatiles, des polymères quaternisés, des hydrolysats protéiniques ou des hydrolysats protéiniques quaternisés.

5. Un procédé selon l'une des revendications précédentes, dans lequel l'agent de conditionnement dans la composition est présent dans une quantité allant de 0,1 à 1 % en masse.

6. Un procédé selon l'une des revendications précédentes, dans lequel l'agent tampon co-acide dans la composition est de l'acide lactique.

7. Un procédé selon l'une des revendications précédentes, dans lequel l'agent tampon co-acide dans la composition est présent dans une quantité allant de 2 à 5% en masse.

8. Un procédé selon l'une des revendications précédentes, dans lequel l'acide 2-hydroxyalcanoïque et l'agent tampon co-acide pris ensemble dans la composition représentent au moins 3 % en masse.

9. L'utilisation, pour améliorer l'élasticité des cheveux, d'une composition aqueuse de conditionnement des cheveux comprenant:

(a) de 0,1 à 20 % en masse d'un acide 2-hydroxyalcanoïque choisi à partir de l'acide 2-hydroxyhexanoïque, de l'acide 2-hydroxyoctanoïque, de l'acide 2-hydroxydécanoïque ou de mélanges de ceux-ci;

(b) de 0,01 à 10 % en masse d'un agent de conditionnement ; et

(c) de 0,1 à 10 % en masse d'un agent tampon co-acide.